(19) Europäisches Patentamt
European Patent Office
Office européen des brevets

(11) Veröffentlichungsnummer: **0 242 551**
**B1**

(12) # EUROPÄISCHE PATENTSCHRIFT

(45) Veröffentlichungstag der Patentschrift: **31.10.90**

(51) Int. Cl.$^5$: **C 07 D 487/04**, C 07 D 229/00

(21) Anmeldenummer: **87103285.0**

(22) Anmeldetag: **07.03.87**

(54) **Verfahren zur Herstellung von Diaziridinen und Produkte daraus.**

(30) Priorität: **11.03.86 DE 3607993**

(43) Veröffentlichungstag der Anmeldung:
**28.10.87 Patentblatt 87/44**

(45) Bekanntmachung des Hinweises auf die Patenterteilung:
**31.10.90 Patentblatt 90/44**

(84) Benannte Vertragsstaaten:
**BE DE FR GB IT NL**

(56) Entgegenhaltungen:
**GB-A- 890 334**
**GB-A- 893 388**

**CHEMISCHE BERICHTE, Band 97, Nr. 1, 1964, Seiten 49-60; E. SCHMITZ et al.: "Diaziridine, VI. Synthese von N.N'-Dialkyl-hydrazinen über Diaziridine"**

**CHEMISCHE BERICHTE, Band 99, Nr. 7, 1966, Seiten 2104-2109; R. OHME et al.: "Diaziridine, VII. Diaziridine aus Aminalen des Formaldehyds"**

(73) Patentinhaber: **BASF Aktiengesellschaft**
**Carl-Bosch-Strasse 38**
**D-6700 Ludwigshafen (DE)**

(72) Erfinder: **Bronstert, Klaus, Dr.**
**Gartenstrasse 26**
**D-6719 Carlsberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 242 551 B1

**Beschreibung**

Die Erfindung betrifft ein Verfahren zur Herstellung von N,N'-Dialkyldiaziridinen oder bicyclischen Diaziridinen der allgemeinen Formeln I oder II:

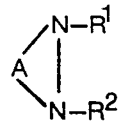

oder

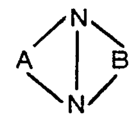

(I) (II)

worin $R^1$ und $R^2$ = ein Alkyl- oder Arylrest, A = eine eingliedrige, gegebenenfalls substituierte Kohlenstoffbrücke und B = eine dreigliedrige, gegebenenfalls substituierte Methylenbrücke, durch Umsetzen eines aliphatischen Mono- oder 1,3-Diamins mit einem aliphatischen cycloaliphatischen, aromatischen oder heterocyclischen Aldehyd oder Keton in Gegenwart eines Halogen enthaltenden Oxidationsmittels.

N,N'-Dialkyldiaziridine oder bicyclische Diaziridine sind Zwischenprodukte zur Herstellung von substituierten Hydrazinen [vgl. Chemische Berichte *97*, (1964), Seiten 49 bis 60] oder dienen zur Ausrüstung von Polymeren mit Aminoendgruppen (vgl. europäische Patentanmeldung 86110539.3). Die bekannten Verfahren zu ihrer Herstellung sind kompliziert, material- und energieaufwendig und liefern unbefriedigende Ausbeuten.

So werden nach dem Stand der Technik mit einem Überschuß von Aminen zunächst Alkylchloramine erhalten, die mit getrennt hergestellten Schiffsche Basen umgesetzt werden. Bei einem anderen Verfahren wird Formaldehyd mit Aminen in Gegenwart eines großen Überschusses Natronlauge umgesetzt und dann bei tiefer Temperatur mit Chlorbleichlauge zur Reaktion gebracht [vgl. Chemische Berichte *99*, (1966), Seiten 2 104 bis 2 109]. Immer liegen die Ausbeuten an den Zielprodukten erheblich unter der Theorie.

Aufgabe der Erfindung ist daher die Verbesserung der Herstellung von N,N'-Dialkylaziridinen und bicyclischen Diaziridinen.

Diese Aufgabe wurde durch ein Verfahren gemäß Patentansprüchen 1 bis 4 gelöst.

In den N,N'-Dialkyldiaziridinen der allgemeinen Formel (I) oder in den bicyclischen Diaziridinen der allgemeinen Formel (II)

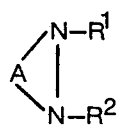

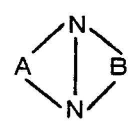

(I) (II)

bedeuten $R^1$ und $R^2$ Alkyl-, insbesondere $C_1$- bis $C_6$-Alkylreste. Besonders bevorzugt ist der Methylrest. Unter A wird eine eingliedrige, gegebenenfalls substituierte Kohlenstoffbrücke verstanden. Bevorzugt ist A eine Methylengruppe der allgemeinen Formel

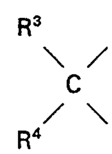

wobei $R^3$ und/oder $R^4$ = Wasserstoff, eine $C_1$- bis $C_4$-Alkylgruppe, eine Phenylgruppe, eine Cyclohexylgruppe, oder wobei $R^3$ und $R^4$ ringförmig geschlossen sind. B bedeutet eine dreigliedrige, gegebenenfalls substituierte Methylenbrücke. Bevorzugt ist B $—CH_2—CH_2—CH_2—$ oder

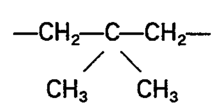

Als Aldehyde bzw. Ketone werden bei dem erfindungsgemäßen Verfahren bevorzugt, Formaldehyd, Acetaldehyd, Isobutylaldehyd, Aceton, Methylethylketon oder Cyclohexan und als Amine bevorzugt Alkylamine mit 1 bis 6 C-Atomen, als Diamine bevorzugt 1,3-Propylendiamin, 2,2-Dimethyl-1,3-propylendiamin und 1,3-Butylendiamin eingesetzt und als Salz der unterchlorigen Säure Natriumhypochlorid verwendet.

Man arbeitet erfindungsgemäß so, daß zunächst die Monoamine mit Aldehyden oder Ketonen im Molverhältnis 2:1 gemsicht werden. Sofern 1,3-Alkylendiamine benützt werden, wird ein Molverhältnis von

1:1 angewandt. Dann wird bei Temperaturen von 25 bis 70, vorzugsweise zwischen 40 und 50°C, unter sehr gutem Rühren eine wäßrige Alkali- oder Erdalkalihypochloridlösung zugetropft (ein Mol aktives Chlor je zwei Mol der eingesetzten amine bzw. ein Mol je Mol Diamin).

Das entstandene Diaziridin wird dann aus der Reaktionslösung mit einem geeigneten Läsungsmittel, bevorzugt mit Chloroform, extrahiert und durch Destillation, bevorzugt unter verringertem Druck, gegebenenfalls durch anschließende Umkristallisierung in reiner Form gewonnen. Im Gegensatz zu den in der Literatur beschriebenen Lösungsmitteln kann auf vorheriges Eindampfen der Reaktionslösung oder Sättigung mit Kaliumhydroxid verzichtet werden.

Bei dem erfindungsgemäßen Verfahren ist insbesondere überraschend, daß die Ausbeute bei Temperaturen zwischen 40 und 50°C ein Optimum erreicht. Unter diesen Bedingungen kann bei der Synthese auf die Verwendung von zusätzlichen Basen wie Aminen oder Alkalihydroxiden verzichtet werden. In der Literatur wird dagegen die Lehre vermittelt, daß in stark alkalischem Medium und bei tiefen Temperaturen zu arbeiten ist [vgl. Chemische Berichte *99*, (1966) Seite 2 105, Zeilen 1 bis 9].

Die Verbesserung der Ausbeute läuft parallel mit der Verminderung oder dem Verschwinden von unerwünschten harzartigen Nebenprodukten, die sich bei bekannten Verfahren während der Reaktion abscheiden und/oder bei der Destillation der Extrakte als Rückstand verbleiben. Erfindungsgemäß ist eine rationellere Herstellung der Diaziridine als nach bekannten Verfahren möglich.

Beispiele 1 und 2

1. Propylen-1,3-diaziridin (1,5-Diazabicyclo-[3,1,0]-hexan)

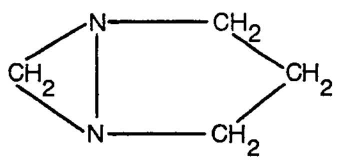

In einem Einliter-Vierhalskolben werden 74 g (1 Mol) 1,3-Diaminopropan vorgelegt. Bei 45°C werden unter Rühren 100 g Formaldehyd (30 gew.%ig), (1 Mol) und anschließend bei gleicher Temperatur unter sehr gutem Rühren 548 $cm^3$ (1 Mol) wäßrige Natriumhypochloritlösung (Gehalt 12,5—13,5 Gew.% aktives Chlor) zugetropft. Nach Abkühlen wird viermal mit je 700 $cm^3$ Chloroform extrahiert. Das Chloroform wird bei leichtem Vakuum abdestilliert (Sumpftemperatur ca. 40°C). Das Diaziridin wird bei 2 mbar abdestilliert. KP = 35—42°C, Rohausbeute 80 g = 95% der Theorie Reiheit 1t. GC* can. 95%.

Das Produkt kann durch Feindestillation mit hohem Rücklaufverhältnis 1-5 über eine Kolonne mit V 2 A-Sulzerpackungen (ϕ = 50 mm, Länge ca. 1 m) zur Vermeidung eines hohen Druckverlustes auf eine Reinheit von mehrmals 99% (GC) gebracht werden. Das Zielprodukt hat einen Siedebereich von ca. 42°C bei 2,0 mb.

Zum Vergleich:

Nach der Vorschrift aus der europäischen Patentanmeldung 86110539.3, Seite 8, Zeilen 5 bis 18 wird eine Rohausbeute von ca. 53% der Theorie erhalten. Als Destillationsrückstand verbleiben ca. 30 g eines klebrigen harzartigen Rückstandes.

2. 2,2-Dimethylpropylen-1,3-diaziridin (1,5-Diaza-3,3-dimethylbicyclo-[3,1,0]hexan)

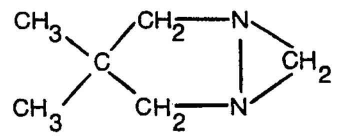

In einem Einliter-Vierhalskolben werden 102 g (1 Mol) 1,3-Diamino-2,2-dimethylpropan vorgelegt. Bei 45°C werden unter Rühren 100 g Formaldehyd 30 gew.%ig und anschließend bei gleicher Temperatur 548 $cm^3$ wäßrige Natriumhypochloridlösung zugetropft.

Nach Abkühlen wir viermal mit 100 $cm^3$ Chloroform extrahiert. Das Chloroform wird bei leichtem Vakuum abdestilliert (Sumpftemperatur = 40°C). Das Diaziridin wird bei 7 mb abdestilliert (KP 60—65°C). Der Kühler der Vakuumdestillation wird dabei, um eine Kristallisation des Diaziridins zu vermeiden, mit 38°C warmem $H_2O$ gekühlt (Thermostat). Die Vorlage wird mit Eiswasser gekühlt, wobei das Destillat zum größten Teil kristallisiert.

Ausbeute ca. 95% der Theorie, Reinheit laut GC* 94—95%.

Reinigung des 2,2-Dimethylpropylen-1,3-diaziridins

Der nach der Destillation angefallene Kristallkuchen wird mit n-Pentan unter trockenem Stickstoff zerdrückt. Nach Abkühlen mit Eiswasser wird der Kristallbrei über eine Glasfilterfritte (Druckfilter) mit trockenem Stickstoff abgedrückt. Der Filterkuchen wird dreimal mit eiskaltem Pentan gewaschen und mit

trockenem Stickstoff bei Raumtemperatur getrocknet.

Aus der Mutterlauge kann durch Einengen weiteres Kristallisat gewonnen werden. Alle Operationen sind unter sorgfältigem Ausschluß von Feuchtigkeit auszuführen.

Bei Einsatz von 968 g Rohware wurden erhalten:

1. Kristallisat = 505 g   Reinh. GC* = 99,4%
2. Kristallisat = 150 g   Reinh. GC* = 99,5%
3. Kristallisat = 71 g   Reinh. GC* = 94,3%

Durch Umkristallisieren des 1. Kristallisates aus Pentan kann ein Diaziridin mit einer Reinheit von 99.73% (GC*) gewonnen werden (FP = 38°C).

Die 3. Mutterlauge enthält noch 77 Gew.% Diaziridin (GC*)

Vergleichsbeispiele

Die Herstellung des 2,2-Dimethylpropylen-1,3-diaziridins wird nach der vorstehenden Vorschrift durchgeführt, mit der Abwandlung, daß die Reaktionstemperaturen gemäß der Tabelle gewählt werden. Neben der Rohausbeute wurde die als Nebenprodukt gebildete Menge eines in der Reaktionsmischung unlöslichen Harzes sowie von harzartigen Destillationsrückständen bestimmt. In weiteren Versuchen wurde das Diamin mit der Formaldehydlösung in Gegenwart von 2-normaler Natronlauge zur Reaktion gebracht. Reaktionsbedingungen und Ausbeuten sind der Tabelle zu entnehmen.

TABELLE 1

| Vergl.-Versuch | 2n NaOH $cm^3$ | Reaktions-temp. °C | Harze g | Rohausbeute Gew.% d. Theorie |
|---|---|---|---|---|
| 1 | 500 | 5 | 34 | 55.3 |
| 2 | 500 | 10 | 33 | 61,3 |
| 3 | 500 | 25 | 18 | 69,4 |
| 4 | 500 | 45 | 0,5 | 78 |
| 5 | — | 45 | — | 90,4 |
| 6 | — | 60 | 11 | 65 |

*zur Herstellung der Gaschromatogramme wurde ein Dani 3800 mit Flammenionisationsdetektor benutzt mit "Fused-Silica"-Kapillare (50 m Länge, ϕ inner 0,25 mm), die mit Silicon SE 54 belegt ist (stationäre Phase).

| | |
|---|---|
| Probengeber-Temperatur: | 140°C |
| Temperaturgradient: | 60°—280°C |
| Heizrate: | 3°C/Min |
| Detektortemperatur: | 350°C |
| Trägergas: | $N_2$ |

Beispiele 3—5

In einem Einliter-Vierhalskolben werden unter gutem Rührem jeweils 2 Mol Amin bzw. 1 Mol Diamin mit den in der Tabelle angegebenen Aldehyden bzw. Ketonen (je 1 Mol) bei 45°C gemischt und dann bei dieser Temperatur 1 Mol wäßrige Natriumhypochloritlösung zugegeben. Bei 20°C wird je dreimal mit Chloroform extrahiert und die entstandenen Diaziridine im Vakuum destillativ gewonnen.

Tabelle 2

| Beispiel | Amin | Aldehyd bzw. Keton | Name und Formel des Diaziridins | Reakt.-temp. °C | Ausbeute | Reinheit lt GC* | KP/mb |
|---|---|---|---|---|---|---|---|
| 3 | 1,3-Diaminopropan | i-Butylaldehyd | 1 | 45 | 96,8 | 98,3 | 33/ 0,1 |
| Vergl.-beispiel | " | " | 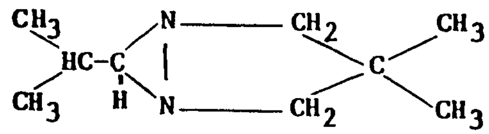 | 5 | 72 | 92,8 | 33-37/ 0,1 |
| 4 | 1,3-Diaminopropan | Aceton | 2 | 45 | 77 | 95 | 37-40/ 0,3 |
| Vergl.-beispiel | " | " | 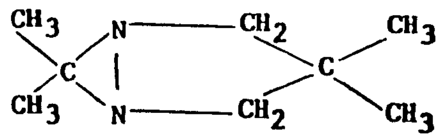 | 5 | 68 | 93 | 37-40/ 0,3 |
| 5 | n-Butylamin | Formaldehyd | 3 | 45 | 80 | 87 | 44-52/ 0,5 |
| Vergl. beispiel | " | " | 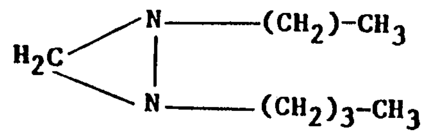 | 5 | 51 | 87 | 44-52/ 0,5 |

1 = 6-i-Propyl-1,5-diazabicyclo[3,1,0]hexan
2 = 6,6-Dimethyl-1,5-diaza-bicyclo[3,1,0]hexan
3 = N,N'-Dibutyldiaziridin (bestehend aus einer Mischung der cistrans-Isomeren)

* Zur Herstellung der Gaschromatogramme wurde ein Dani 3800 mit Flammenionisationsdetektor benutzt mit "Fused-Silica"-Kapillare (50 m Länge, Ø innen 0,25 mm), die mit Silicon SE 54 belegt ist (Stationäre Phase).

| | |
|---|---|
| Probengeber-Temperatur : | 140°C |
| Temperaturgradient: | 60°-280°C |
| Heizrate: | 3°C/Min |
| Detektortemperatur: | 350°C |
| Trägergas: | $N_2$ |

## Patentansprüche

1. Verfahren zur Herstellung von N,N'-Dialkyldiaziridinen oder bicyclischen Diaziridinen der allgemeinen Formeln I bzw. II

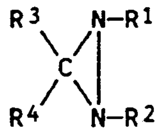 (I) 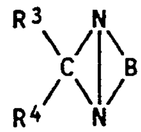 (II)

worin $R^1$ und $R^2$ je einen $C_1$—$C_6$-Alkylrest, $R^3$ und $R^4$ Wasserstoff, $C_1$—$C_4$-Alkyl, Phenyl oder Cyclohexyl bedeuten und B den zweiwertigen Rest $-(CH_2)_3-$ oder $—CH_2—C(CH_3)_2—CH_2—$ bedeutet, durch Umsetzen eines entsprechenden aliphatischen Mono- oder 1,3-Diamins mit einem aliphatischen, cycloaliphatischen, aromatischen oder heterocyclischen Aldehyd oder Keton in Gegenwart eines Salzes der unterchlorigen Säure, dadurch gekennzeichnet, daß eine Mischung aus zwei Mol des Monoamins oder ein Mol des 1,3-Diamins mit einem Mol des Aldehyds oder Ketons bei Temperaturen zwischen 25 und 70°C unter guter Durchmischung mit einem Mol der wäßrigen Lösung eines Alkali- oder Erdalkalisalzes der unterchlorigen Säure zur Reaktion gebracht wird und dann mit einem organischen Lösungsmittel aus der Reaktionsmischung extrahiert und nach bekannten Verfahren aufdestilliert oder gereinigt wird.

2. Verfahren nach Anspruch 1, dadurch gekennzeichnet, daß die Temparatur zwischen 40 und 50°C eingestellt wird.

3. Verfahren nach Anspruch 1, dadurch gekennzeichnet, daß als Salz der unterchlorigen Säure Natriumhypochlorit eingesetzt wird.

4. Verfahren nach Anspruch 1, dadurch gekennzeichnet, daß das organische Lösungsmittel Chloroform ist.

## Revendications

1. Procédé de préparation de N,N'-dialkyldiaziridines ou de diaziridines bicycliques répondant aux formules générales I ou II

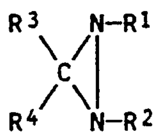 (I) 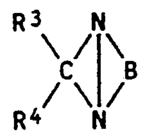 (II)

dans lesquelles $R^1$ et $R^2$ représentent chacun un radical alkyle en $C_1$—$C_6$, $R^3$ et $R^4$ représentent des atomes d'hydrogène, des radicaux alkyle en $C_1$—$C_4$, phényle ou cyclohexyle et B représente le radical bivalent $-(CH_2)_3-$ ou $—CH_2—C(CH_3)_2—CH_2—$ par la réaction d'une mono- ou 1,3-diamine aliphatique correspondante avec un aldéhyde ou une cétone aliphatique, cycloaliphatique, aromatique ou hétérocyclique, en présence d'un sel de l'acide hypochloreux, caractérisé en ce que l'on fait réagir un mélange constitué de deux moles de la monoamine ou d'une mole de la 1,3-diamine et d'une mole de l'aldéhyde ou de la cétone, à des températures variant de 25 à 70°C et sous bon mélange intime, avec une mole de la solution aqueuse d'un sel de métal alcalin ou de métal alcalino-terreux de l'acide hypochloreux et on extrait ensuite le mélange réactionnel avec un solvant organique et on purifie ensuite le produit par distillation selon des procédés connus.

2. Procédé suivant la revendication 1, caractérisé en ce que la température varie de 40 à 50°C.

3. Procédé selon la revendication 1, caractérisé en ce que l'on utilise l'hypochlorite de sodium à titre de sel de l'acide hypochloreux.

4. Procédé suivant la revendication 1, caractérisé en ce que le solvant organique est le chloroforme.

## Claims

1. A process for preparing an N,N'-dialkyldiaziridine or bicyclic diaziridine of the general formulae I and II

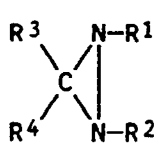 (I) 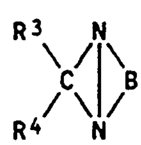 (II)

where $R^1$ and $R^2$ are each $C_1$—$C_6$-alkyl, $R^3$ and $R^4$ are each hydrogen, $C_1$—$C_4$-alkyl, phenyl or cyclohexyl and B is the divalent radical $-(CH_2)_3-$ or $—CH_2—C(CH_3)_2—CH_2—$, by reacting an appropriate aliphatic

monoamine or 1,3-diamine with an aliphatic, cycloaliphatic, aromatic or heterocyclic aldehyde or ketone in the presence of a hypochlorous acid salt, which comprises reacting a mixture of 2 moles of the monoamine or 1 mole of the 1,3-diamine with one mole of the aldehyde or ketone at 25—70°C while thoroughly mixing with 1 mole of an aqeuous solution of an alkali metal or alkaline earth metal salt of hypochlorous acid and then extracting with organic solvent from the reaction mixture and distilling or purifying in a conventional manner.

2. A process as claimed in claim 1, wherein the temperature is set within the range from 40 to 50°C.

3. A process as claimed in claim 1, wherein the hypochlorous acid salt used is sodium hypochlorite.

4. A process as claimed in claim 1, wherein the organic solvent is chloroform.